(19) Europäisches Patentamt
European Patent Office
Office européen des brevets

(11) EP 3 012 757 B1

(12) **EUROPEAN PATENT SPECIFICATION**

(45) Date of publication and mention of the grant of the patent:
**25.12.2019 Bulletin 2019/52**

(51) Int Cl.:
***G16H 20/30*** *(2018.01)*

(21) Application number: **15190693.0**

(22) Date of filing: **21.10.2015**

# (54) EXERCISE MANAGING METHOD AND APPARATUS

ÜBUNGSVERWALTUNGSVERFAHREN UND -VORRICHTUNG

PROCÉDÉ ET APPAREIL DE GESTION D'EXERCICE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **22.10.2014 KR 20140143176**

(43) Date of publication of application:
**27.04.2016 Bulletin 2016/17**

(73) Proprietors:
- **Samsung Electronics Co., Ltd.**
  **Gyeonggi-do 443-742 (KR)**
- **University-Industry Cooperation Group of Kyung Hee University**
  **Gyeonggi-do 446-701 (KR)**

(72) Inventors:
- **KO, Byunghoon**
  **443-803 Gyeonggi-do (KR)**
- **BAE, SangKon**
  **443-803 Gyeonggi-do (KR)**
- **LEE, Chonghee**
  **443-803 Gyeonggi-do (KR)**
- **SUNOO, Sub**
  **446-701 Gyeonggi-do (KR)**
- **NAM, Sang Seok**
  **446-701 Gyeonggi-do (KR)**
- **PARK, Hun Young**
  **446-701 Gyeonggi-do (KR)**

(74) Representative: **Grünecker Patent- und Rechtsanwälte**
**PartG mbB**
**Leopoldstraße 4**
**80802 München (DE)**

(56) References cited:
**EP-A1- 1 510 175 EP-A2- 1 369 082**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 012 757 B1

## Description

1. Field

The following description relates to a method and apparatus that analyze and evaluate characteristics of an exercise performed by a user.

2. Description of Related Art

An exercise prescription assists a user in achieving desired exercise effects by suggesting an exercise frequency, an exercise intensity, an exercise duration, and an exercise type to the user. For example, the user may desire to increase the heart rate to a certain level, lose 10 pounds in three months, become fit to run a marathon in six months, or obtain more toned muscles. These effects are examples of desired exercise effects. An exercise frequency refers to the frequency with which an exercise is recommended to be performed by the user. An exercise intensity refers to the difficulty level of the exercise. An exercise duration refers to how long the exercise should be performed. An exercise type refers to a kind of activity that is performed as the exercise.

An exercise quantity is a factor significantly affecting the achievement of the exercise effects. Thus, accurate and quantitative calculation of the exercise quantity may be considerably significant in an exercise prescription. The exercise quantity may be determined based on the exercise intensity and the exercise duration. While the exercise duration is quantitatively measurable, the exercise intensity are generally expressed in terms of restricted measurable indices. For example, the exercise intensity may be expressed by an oxygen uptake per unit time. However, to measure an oxygen uptake, expensive equipment and professional analysis may be required. Thus, such measurement may be inaccessible to the user.

EP 1 369 082 A2 discloses a physical activity measurement apparatus, wherein a CPU calculates the user's heart rate while exercising from a pulse wave signal output from a pulse wave sensor unit.

EP 1 510 175 A1 discloses an exercise manager program for adapting parameters of a training device.

## SUMMARY

It is therefore the object of the present invention to provide an improved exercise managing method, a corresponding exercise managing apparatus, and a corresponding non-transitory computer-readable storage medium.

This object is solved by the subject matter of the independent claims.

Preferred embodiments are defined by the dependent claims.

This summary is provided to introduce a selection of concepts in a simplified form that are further described below in the Detailed Description.

In one general aspect, there is provided an exercise managing method involving determining, with a processor, a cumulative heart rate (HR) indicating a cumulative sum of HRs of a user, analyzing an exercise performed by the user based on the cumulative HR, and providing a result of the analyzing to the user.

The cumulative HR includes at least one of a first cumulative HR calculated based on HRs measured from a start time of the exercise, and a second cumulative HR based on HRs during a steady state.

The analyzing comprises determining a ratio of anaerobic metabolism to aerobic metabolism used by the user during a time period of the exercise, based on a difference between the second cumulative HR and the first cumulative HR or determining an exercise performance excess or shortfall ratio based on a difference between the cumulative HR and a target cumulative HR for the user, wherein the target cumulative HR is determined based on a target HR for the user and the duration of the exercise.

The determining may involve determining the first cumulative HR by continuously accumulating HRs of the user from the start time of the exercise.

The determining may involve determining the second cumulative HR based on a mean HR in the steady state and a duration of the exercise.

In another general aspect, there is provided a non-transitory computer-readable storage medium storing instructions to cause a computer to perform the general aspect of the method described above.

In yet another general aspect, there is provided an exercise managing apparatus including a heart rate (HR) measurer configured to measure a change in an HR of a user, an exercise manager configured to determine a cumulative HR in an exercise period of the user based on a mean HR in a steady state period and a duration of an exercise performed by the user, and analyze an exercise performed by the user based on the determined cumulative HR, and a display unit configured to display a result of the analyzing.

The analyzing of the exercise comprises: determining a ratio of anaerobic metabolism to aerobic metabolism used by the user during a time period of the exercise, based on a difference between the cumulative HR and another cumulative HR, wherein the another HR is calculated based on HRs measured from a start time of the exercise performed by the user; or determining an exercise performance excess or shortfall ratio based on a difference between the cumulative HR and a target cumulative HR for the user, wherein the target cumulative HR is determined based on a target HR for the user and the duration of the exercise.

The exercise manager may be configured to determine that the HR reaches the steady state in response to the change in the HR of the user or an elapsed time of the exercise satisfies a predetermined condition.

## BRIEF DESCRIPTION OF THE DRAWINGS

FIG. 1 illustrates an example of a method of operating an exercise managing apparatus.
FIG. 2 illustrates another example of a method of operating an exercise managing apparatus.
FIG. 3 is a block diagram illustrating an example of a configuration of an exercise managing apparatus.
FIG. 4 is a block diagram illustrating another example of a configuration of an exercise managing apparatus.
FIG. 5 illustrates an example of a method of analyzing characteristics of an exercise of a user based on a change in a heart rate (HR) of the user using an exercise managing apparatus.
FIG. 6 illustrates an example of a method of analyzing characteristics of an exercise based on a change in an HR using an exercise managing apparatus in a case in which a user performs an exercise at various intensities.
FIG. 7A illustrates an example of an exercise managing apparatus displaying a result of evaluating exercise effects.
FIG. 7B illustrates an example of an exercise managing apparatus displaying a result of evaluating exercise effects.
FIG. 8 is a flowchart illustrating an example of an exercise managing method.

Throughout the drawings and the detailed description, unless otherwise described or provided, the same drawing reference numerals will be understood to refer to the same elements, features, and structures. The drawings may not be to scale, and the relative size, proportions, and depiction of elements in the drawings may be exaggerated for clarity, illustration, and convenience.

## DETAILED DESCRIPTION

The following detailed description is provided to assist the reader in gaining a comprehensive understanding of the methods, apparatuses, and/or systems described herein. However, various changes, modifications, and equivalents of the systems, apparatuses and/or methods described herein will be apparent to one of ordinary skill in the art.

The features described herein may be embodied in different forms, and are not to be construed as being limited to the examples described herein. The invention is defined by the appended claims. The examples, aspects and embodiments not falling within the scope of the claims are for reference only.

FIG. 1 illustrates an example of a method of operating an exercise managing apparatus.

The exercise managing apparatus may analyze or evaluate an exercise performed by a user. Further, the exercise managing apparatus may suggest an exercise objective to the user, and may manage exercise performance records and exercise schedules that are personal to the user. The exercise managing apparatus may adjust the exercise objective set for the user based on a result of analyzing the exercise performed by the user, and may guide the user to an optimized exercise objective or exercise course.

The exercise managing apparatus may analyze the exercise performed by the user based on heart rate (HR) information measured while the user is performing the exercise. The HR information may be measured using a sensor embedded in the exercise managing apparatus or with an external sensor that detects one or more biosignal of the user; however, the present disclosure is not limited thereto. The exercise managing apparatus may provide the result of analyzing the exercise to the user, and set the optimized exercise objective or exercise course for the user based on the result of analyzing. The analyzing may involve the use of one or more computer processor and memory storage found in the exercise managing apparatus.

To achieve desired exercise effects, accurate calculation of the exercise quantity may be significant. The exercise quantity may be determined based on the exercise intensity and the exercise duration, and the exercise intensity may be determined based on an HR. In general, the higher the exercise intensity, the higher the HR used to perform the exercise at the corresponding exercise intensity. Thus, a high HR detected while an exercise is being performed may indicate a high intensity of the exercise being performed.

The exercise managing apparatus may determine a total exercise quantity based on HRs measured at a body of a user. The exercise managing apparatus may determine a cumulative HR based on HR information of the user, and analyze the exercise performed by the user based on the cumulative HR. The cumulative HR may refer to a cumulative sum of HRs reflecting an exercise intensity for each time period.

The exercise managing apparatus may determine the total quantity of the exercise performed by the user and a quantity of the exercise performed at various exercise intensities based on the cumulative HR and store the quantities in a memory of the exercise managing apparatus. In addition, the exercise managing apparatus may determine a ratio of anaerobic metabolism to aerobic metabolism used during the exercise based on the cumulative HR. Metabolism refers to a series of chemical reactions that occur in the body to generate energy used for an exercise. The aerobic metabolism may require oxygen to generate energy, whereas the anaerobic metabolism may not require oxygen.

According to one example, the exercise managing apparatus may be embedded in and may operate within a wearable device 110. In the example illustrated in FIG. 1, the wearable device 110 is a wrist-worn device provided in a form of a watch or a bracelet. When a user 120 exercises while wearing the wearable device 110, the exercise managing apparatus may analyze with a processor the characteristics of the exercise performed by the user 120 based on HR information measured at a wrist of the user 120. The HR information may be measured with a sensor provide on a bottom surface of the wearable device 110 that contacts the user.

The exercise managing apparatus may provide a result of analyzing the exercise to the user 120. For example, the exercise managing apparatus may display a result of analyzing an exercise currently being performed and preset exercise objective information. In addition, the exercise managing apparatus may indicate to the user 120 that a predetermined condition related to the exercise is achieved, through an audio signal, oscillation, or a change in brightness of a display. For example, when a preset target exercise duration elapses or when a target exercise quantity is achieved, the exercise managing apparatus may output a pre-stored audio signal through a speaker, generate oscillation, or continually change a brightness of the display. The wearable device 110 may include a display screen, a microphone, a speaker or other input/output device.

The wearable device 110 including the exercise managing apparatus may interoperate with a terminal device 130 via a transmitter and a receiver, and may share data with the terminal device 130. For example, the result of analyzing the exercise or the HR signals measured at the wrist of the user 120 may be transmitted to the terminal device 130. In addition, the terminal device 130 may transmit, to the exercise managing apparatus, exercise objective data or exercise record data related to previously performed exercises. The terminal device 130 may store the result of analyzing the exercise received from the exercise managing apparatus in a memory of the terminal device 130, and may manage the exercise record data and the exercise objective data of the user 120 through exercise contents.

In another example, the exercise managing apparatus may be embedded and operate in the terminal device 130. The wearable device 110 may be worn on the wrist of the user 120, and measure HR signals of the user 120 at the wrist. The wearable device 110 may amplify and filter the measured HR signals, or convert the analog HR signals into digital signals. The wearable device 110 may transmit the converted digital HR signals to the terminal device 130. The exercise managing apparatus included in the terminal device 130 may analyze characteristics of the exercise performed by the user 120 based on the HR information received from the wearable device 110. The exercise managing apparatus may display a result of the analyzing on a display screen or store the result in a memory. In addition, the exercise managing apparatus may manage exercise record data and exercise objective data of the user 120 through exercise contents.

Hereinafter, an example of a method of analyzing and evaluating an exercise performed by a user based on HR information using an exercise managing apparatus will be described in detail.

FIG. 2 illustrates another example of a method of operating an exercise managing apparatus 220.

An HR sensing device 210 may measure HR signals at a body of a user. The HR sensing device 210 may measure the HR signals by sensing potential signals of a body part at which heartbeats of the user are found. For example, the HR sensing device 210 may include a sensor that measures the HR signals of the user at a chest, finger tips, a wrist, or a forearm of the user. HR signals measured while the user is exercising may be transmitted to the exercise managing apparatus 220.

The exercise managing apparatus 220 may analyze or evaluate an exercise performed by the user based on the HR information received from the HR sensing device 210. The exercise managing apparatus 220 may determine a cumulative HR indicating a cumulative sum of HRs based on the HR information, and analyze the exercise of the user based on the cumulative HR. The exercise managing apparatus 220 may evaluate, based on the cumulative HR, a total quantity of the exercise performed by the user, a usage ratio of anaerobic metabolism to aerobic metabolism, or whether an exercise objective is achieved. For descriptions of the exercise managing apparatus 220, reference may be made to descriptions of an exercise managing apparatus 310 of FIG. 3 or an exercise managing apparatus 410 of FIG. 4.

A result of analyzing characteristics of the exercise may be provided to the user through an interface device connected to the exercise managing apparatus 220, or transmitted to a server 230. The exercise managing apparatus 220 may transmit the result of analyzing the exercise to the server 230 through networks including, for example, the Internet, an intranet, a wired and wireless communications network, or a mobile communications network.

The server 230 may receive exercise record data, exercise analysis data, or personal data of the user from the exercise managing apparatus 220. The exercise record data may include record information related to exercises previously performed by the user, and the exercise analysis data may include information about a result of analyzing exercises previously performed by the user. The personal data may include information about a gender, an age, a height, and a weight of the user.

The server 230 may store and manage data received from the exercise managing apparatus 220, and transmit data requested by the exercise managing apparatus 220 to the exercise managing apparatus 220. The server 230 may include a memory, one or more processor, a transmitter, a receiver and the like. The server 230 may manage exercise records of the user through exercise contents, and set an exercise guide such as, for example, an exercise objective or an exercise course suitable for the user. The exercise guide set by the server 230 may be transmitted to the exercise managing apparatus 220, and the exercise managing apparatus 220 may guide the user to perform an exercise based on the exercise guide.

FIG. 3 is a block diagram illustrating an example of a configuration of the exercise managing apparatus 310.

Referring to FIG. 3, the exercise managing apparatus 310 includes an interface unit 320 and an exercise manager 330. An operation of the exercise manager 330 may be performed by at least one processor. The interface unit may include at least one processor and may communicate with a sensing device, a sensor, a camera and the like.

For example, the interface unit 320 may receive HR information measured at a body of a user. The interface unit 320 may receive HR information transmitted from an HR sensing device (not shown), and transmit the received HR information to the exercise manager 330. The exercise manager 330 may include one or more computer processor.

The exercise manager 330 may analyze characteristics of an exercise performed by the user based on the HR information. The exercise manager 330 may determine an intensity of the exercise performed by the user based on the HR information. In general, an HR increases in proportion to an exercise intensity. The exercise manager 330 may determine the intensity of the exercise performed by the user based on the foregoing characteristic.

The exercise manager 330 may determine a cumulative HR indicating a cumulative sum of HRs based on the HR information, and analyze the exercise of the user based on the determined cumulative HR.

The exercise manager 330 may determine a first cumulative HR based on actual HRs measured from a start time of the exercise. The exercise manager 330 may determine the first cumulative HR by continuously accumulating HRs measured from a point in time at which an HR starts to increase after the user initiates an exercise. For example, the exercise manager 330 may determine the first cumulative HR by summing up the actual HRs measured from the start time of the exercise to an end time of the exercise.

The exercise manager 330 may determine a quantity of the exercise performed by the user based on the first cumulative HR. An exercise quantity may be determined based on an exercise intensity and an exercise duration, and the exercise intensity may be determined based on HRs. A value of the first cumulative HR corresponding to the cumulative sum of the actual HRs may correspond to a value of the total quantity of the exercise performed by the user.

The exercise manager 330 may determine a second cumulative HR in an exercise period of the user based on an HR in a steady state. The exercise manager 330 may determine the second cumulative HR based on a mean HR in a steady state period and a duration of the exercise. For example, the exercise manager 330 may determine a value obtained by multiplying the mean HR in the steady state period by the total duration of the exercise to be the second cumulative HR. The steady state period may refer to a time period during which a steady state is reached.

The exercise manager 330 may determine the HR in the steady state based on the HR information. The steady state may refer to a state in which an HR of the user gradually increases and is maintained almost uniformly without great changes after the user initiates an exercise. The exercise manager 330 may determine that the HR reaches the steady state when a change in the HR of the user or an elapsed time of the exercise satisfies a preset condition. For example, the exercise manager 330 may determine that the HR of the user reaches the steady state when a predetermined time period elapses after the exercise is initiated, or when an increment in the HR decreases to be within a predetermined range after the exercise is initiated.

The exercise manager 330 may determine a ratio of anaerobic metabolism to aerobic metabolism used while the user is exercising, based on the first cumulative HR and the second cumulative HR. The exercise manager 330 may determine the usage ratio of the anaerobic metabolism during the exercise period during which the user performs the exercise based on a difference between the second cumulative HR and the first cumulative HR. Conversely, a value obtained by subtracting the difference between the second cumulative HR and the first cumulative HR from the second cumulative HR may correspond to an exercise quantity related to the aerobic metabolism.

For an aerobic metabolism that produces energy using oxygen to occur during an exercise, an oxygen uptake sufficient to produce the energy required during the exercise may be required. When a user initiates an exercise in a rest state, the oxygen uptake may start to gradually increase to keep up with the energy use of the body. However, the user may not immediately take in an amount of oxygen sufficient to satisfy an amount of oxygen demand by the exercising body. In general, a steady state in which an amount of oxygen demand equals to an oxygen uptake may be reached within about five minutes after an exercise is initiated. In such a steady state, aerobic metabolism may be enabled. A state before the steady state is reached may be referred to as an oxygen deficit state. In the oxygen deficit state, energy required for an exercise may be generated by anaerobic metabolism.

The second cumulative HR determined based on the HR in the steady state may indicate that the entire exercise is performed through both aerobic metabolism and anaerobic metabolism. Conversely, the first cumulative HR corresponding to a cumulative value of actual HRs measured after the exercise is initiated may include a portion corresponding to the aerobic metabolism, excluding an oxygen deficit state after the exercise is initiated and before the steady state is reached. Thus, the difference between the second cumulative HR and the first cumulative HR may reflect a portion of the exercise performed by the anaerobic metabolism, rather than the aerobic metabolism.

The user may receive exercise objective data in relation to an exercise to be performed from the exercise managing apparatus 310. For example, the user may receive an exercise objective such as an exercise type, an exercise duration, an exercise intensity, and an exercise intensity for an exercise duration, for example.

The exercise manager 330 may evaluate a result of performing the exercise based on exercise objective data set by the user. The exercise manager 330 may determine whether HRs measured while the user is exercising reaches a target HR, or whether a cumulative HR calculated during the exercise reaches a target cumulative HR. The target cumulative HR may indicate a demand for a cumulative HR with respect to the target HR.

The exercise manager 330 may evaluate the exercise of the user based on the target cumulative HR and the second cumulative HR determined based on the HR in the steady state, and may determine a result of the evaluating. The result of the evaluating may include an exercise performance excess or shortfall rate indicating an excess portion or a shortfall portion of the quantity of the exercise performed by the user with respect to a target exercise quantity. The exercise manager 330 may calculate a difference between the second cumulative HR and the target cumulative HR after the steady state is reached, and determine the exercise performance excess or shortfall rate based on a ratio of the second cumulative HR after the steady state is reached to the difference.

When the user performs an exercise at a uniform intensity in the entire exercise period, an HR may be maintained uniformly without great changes after reaching a steady state. Conversely, the user may complexly perform an exercise at various intensities. In this example, an HR may be out of a steady state at a point in time an exercise intensity changes, and increase or decrease until another steady state is reached. For example, when a user increases an exercise intensity to a speed of 10 kilometers per hour (km/h) while running at a speed of 8 km/h, an HR of the user may be in a steady state first, start to increase from the point in time at which the user started to run at the speed of 10 km/h, and reach a new steady state.

The cumulative HR may be used to analyze or evaluate an exercise having a uniform intensity and an exercise having various intensities as described above. When the user performs an exercise at various intensities, the exercise manager 330 may divide the entire exercise period into a plurality of exercise periods based on the HR in the steady state and the respective exercise intensities, and determine the first cumulative HR or the second cumulative HR in each of the divided exercise periods. The exercise manager 330 may determine the first cumulative HR in the entire exercise period by summing up first cumulative HRs in the respective exercise periods, or determine the first cumulative HR with respect to the entire exercise period by continuously accumulating actual HRs measured from a start time of the exercise. The exercise manager 330 may determine the second cumulative HR in the entire exercise period by summing up second cumulative HRs in the respective exercise periods. The exercise manager 330 may analyze the exercise performed by the user based on the first cumulative HR and the second cumulative HR determined with respect to the entire exercise period, and output a result of the analyzing.

The exercise manager 330 may compare exercise analysis result data to preset exercise objective data, and generate exercise evaluation data including evaluation information of the exercise performed by the user. The exercise manager 330 may provide the exercise analysis result data and the exercise evaluation data to the user.

The exercise evaluation data may include, for example, evaluation information regarding whether a total quantity of the exercise performed by the user reaches a target exercise quantity, or whether the exercise is performed continuously during a target exercise duration. The exercise objective data may include data related to at least one of an exercise type to be performed by the user, a target exercise quantity, a target HR or target exercise intensity, and a target exercise duration. In an example, the exercise objective data may be set directly by an input provided by the user. For example, the user may directly set an exercise type desired to be performed, a target exercise duration, or a target exercise quantity.

The exercise manager 330 may update the exercise objective data set for the user based on a result of analyzing the exercise. The exercise manager 330 may determine whether a preset exercise objective is achieved as a result of performing the exercise, and may determine whether an exercise objective set for the user is to be adjusted.

For example, when the exercise objective is achieved as the result of performing the exercise, the exercise manager 330 may upgrade the exercise objective, for example, increase the exercise intensity or the exercise duration for an exercise to be performed later by the user. Conversely, when the exercise objective is not achieved as the result of performing the exercise, the exercise manager 330 may downgrade the exercise objective, for example, decrease the exercise intensity or the exercise duration for an exercise to be performed later by the user.

The exercise managing apparatus 310 may set an exercise objective suitable for characteristics for each user based on HR information detected in exercise periods of users, and evaluate exercises performed by the users through quantification.

FIG. 4 is a block diagram illustrating another example of a configuration of an exercise managing apparatus 410.

Referring to FIG. 4, the exercise managing apparatus 410 includes an HR measurer 420, an exercise manager 430, a display unit 440 and a data storage 450. The exercise managing apparatus 410 includes one or more sensor, and the exercise manager 430 includes at least one processor. An operation of the exercising manager 430 may be performed by at least one processor. The processor may store or retrieve information from the data storage 450.

According to one example, the HR measurer 420 may be attached to a body of a user, and may measure HR signals of the user during an exercise period of the user. The HR measurer 420 may measure the HR signals at the body of the user using an electromyography (EMG) sensor or an HR measuring sensor configured to sense potential signals at the body of the user. The HR measurer 420 may transmit HR information measured at the body of the user to the exercise manager 430.

The exercise manager 430 may include at least one processor and may analyze the exercise performed by the user based on the HR information. The exercise manager 430 may determine a cumulative HR indicating a cumulative sum of HRs based on the HR information, and analyze characteristics of the exercise performed by the user based on the determined cumulative HR. The cumulative HR may include a first cumulative HR determined based on actual HRs measured from a start time of the exercise, and a second cumulative HR determined based on an HR in a steady state.

The exercise manager 430 may determine the first cumulative HR by continuously accumulating HRs measured from a point in time at which an HR starts to increase after the user initiates the exercise. The exercise manager 430 may determine a total quantity of the exercise performed by the user based on the first cumulative HR.

The exercise manager 430 may determine the second cumulative HR in the exercise period of the user based on the HR in the steady state. When a change in the HR of the user or an elapsed time of the exercise satisfies a preset condition, the exercise manager 430 may determine that the HR of the user reaches the steady state. The exercise manager 430 may determine the second cumulative HR based on a mean HR in a steady state period and a duration of the exercise.

The exercise manager 430 may determine a ratio of anaerobic metabolism to aerobic metabolism used during the exercise of the user based on the first cumulative HR and the second cumulative HR. The exercise manager 430 may determine the ratio of the anaerobic metabolism used in a time period during which the exercise is performed based on a difference between the second cumulative HR and the first cumulative HR.

The exercise manager 430 may evaluate the exercise performed by the user based on exercise objective data set for the user. The exercise manager 430 may compare exercise analysis result data to the preset exercise objective data, and generate exercise evaluation data including evaluation information of the exercise performed by the user. The exercise objective data may include at least one of an exercise type, a target exercise quantity, an exercise intensity, and a target exercise duration.

The exercise manager 430 may determine a result of evaluating the exercise based on the second cumulative HR determined based on the HR in the steady state and a target cumulative HR. For example, the exercise manager 430 may calculate a difference between the second cumulative HR after the steady state is reached and the target cumulative HR, and determine an exercise performance excess or shortfall rate based on a ratio of the second cumulative HR after the steady state is reached to the difference. The exercise manager 430 may update the exercise objective data set for the user based on a result of analyzing the exercise. For descriptions of the exercise manager 430, reference may be made to the descriptions of the exercise manager 330 of FIG. 3.

The display unit 440 may display the result of analyzing the exercise performed by the user. The display unit 440 may display the exercise objective data set for the user and the exercise evaluation data. For example, the display unit 440 may display a target HR for the user, a target cumulative HR, a currently measured HR, a cumulative HR determined until a current exercise time, an exercise performance excess rate, or a usage ratio of anaerobic metabolism to aerobic metabolism. The display unit 440 includes a hardware component such as a touch screen, a display screen, an LCD display screen, a LED display screen, an OLED display screen, a projector for projecting an image on a screen, or the like.

FIG. 5 illustrates an example of a method of analyzing characteristics of an exercise of a user based on a change in an HR of the user using an exercise managing apparatus.

A graph of FIG. 5 illustrates a change in the HR of the user for approximately thirty minutes from a start time of the exercise to an end time of the exercise. As shown in the graph, the HR does not immediately reach the target HR required to maintain the normal exercise level upon initiation of the exercise. Rather, the HR gradually increases with a bit of delay time until the HR reaches a steady state.

For example, when a user initiates running at a speed of 8 km/h on exercise equipment such as a treadmill, an HR of the user may gradually increase from an HR measured in a rest state, reach an HR suitable for maintaining the speed of 8 km/h, and be maintained uniformly without great changes in a subsequent time period. A state in which an HR is maintained uniformly without great changes from a predetermined time period after an exercise is initiated may be referred to as the steady state.

The exercise managing apparatus may determine various parameters to be used to evaluate the exercise of the user based on a change in the HR occurring during the exercise of the user. The exercise managing apparatus may evaluate the exercise performed by the user based on the determined parameters. The exercise managing apparatus may selectively determine parameters to be used based on evaluation items.

A target HR refers to an HR assumed to be measured in a steady state during an exercise, and may be predetermined for a user. For example, the target HR may be an HR to be achieved by the user in the steady state to achieve exercise effects suggested in an exercise prescription. A resting HR refers to an HR in a rest state before the user initiates an exercise. In the example illustrated in FIG. 5, it is assumed that the resting HR is 60 beats per minute (bpm).

A target cumulative HR refers to a demand for a cumulative HR with respect to a target HR. The target cumulative HR may be used to determine a sum of an aerobic exercise quantity and an anaerobic exercise quantity based on the target HR. The target cumulative HR may be determined by multiplying a target HR by an exercise duration, as expressed by, for example, Equation 1.

[Equation 1]

$$\text{Target cumulative HR} = \text{Target HR} \times \text{Exercise duration}$$

A measured HR refers to an actual HR measured during an exercise of a user. $HR_{steady}$ refers to an HR in a steady state. In the example illustrated in FIG. 5, it is assumed that the HR of the user reaches the steady state within a maximum of five minutes. For example, the exercise managing apparatus may determine a mean HR in a steady state period to be $HR_{steady}$.

A first cumulative HR refers to a cumulative sum of actual HRs measured in an exercise period of the user. A total quantity of the exercise actually performed by the user may be determined based on the first cumulative HR. The first cumulative HR may be determined by summing up all HRs measured from a start time of the exercise to an end time of the exercise, as expressed by, for example, Equation 2.

[Equation 2]

$$\text{First cumulative HR} = \sum_{i=start}^{end} \text{Measured HR}_i$$

A second cumulative HR refers to a cumulative HR determined based on an HR in the steady state. The second cumulative HR may be used to determine a sum of an aerobic exercise quantity and an anaerobic exercise quantity based on the HR in the steady state. The second cumulative HR may be determined by multiplying $HR_{steady}$ by an exercise duration, as expressed by, for example, Equation 3.

[Equation 3]

$$\text{Second cumulative HR} = HR_{steady} \times \text{Exercise duration}$$

$\Delta HR_{steady}$ refers to a difference between measured $HR_{steady}$ corresponding to an HR measured in the steady state period and a target HR, as expressed by Equation 4.

[Equation 4]

$$\Delta HR_{steady} = \text{Measured } HR_{steady} - \text{Target HR}$$

$\Delta HR_{steady}$ may be used as an evaluation index to evaluate whether the quantity of the actually performed exercise is greater than, equal to, or less than an exercise objective set for the user.

$\Delta HR\ Sum_{steady}$ refers to a sum of $\Delta HR_{steady}$s. $\Delta HR\ Sum_{steady}$ may be determined by summing up $\Delta HR_{steady}$s from a start time of the steady state to the end time of the exercise, as expressed by Equation 5.

[Equation 5]

$$\Delta HR\ Sum_{steady} = \sum_{i=steady\ state}^{end} \Delta HR_{steady}$$

$\Delta$HR $Sum_{steady}$ may be used as an evaluation index to evaluate whether a quantity of an exercise actually performed after the steady state is reached is greater than, equal to, or less than a target exercise quantity, in comparison to the exercise objective after the user ends the exercise. $\Delta$HR $Sum_{steady}$ may be determined based on the measured HR in the steady state period. Thus, $\Delta$HR $Sum_{steady}$ may reflect a portion related to an aerobic exercise.

$HR_{deficit}$ refers to a portion corresponding to an exercise quantity related to anaerobic metabolism during the actual exercise. $HR_{deficit}$ may be a difference between the second cumulative HR and the first cumulative HR, as expressed by Equation 6.

[Equation 6]

$$HR_{deficit} = \text{Second cumulative HR - First cumulative HR}$$

$HR_{deficit}$ may be used to determine a usage degree of the anaerobic metabolism during the exercise. The exercise managing apparatus may determine a ratio of $HR_{deficit}$ to the second cumulative HR to be a ratio of the anaerobic metabolism used in the exercise period. A usage ratio of aerobic metabolism may be obtained by subtracting the ratio of the anaerobic metabolism from the entire ratio.

Hereinafter, an example of a method of analyzing an exercise of a user using an exercise managing apparatus based on the HR information shown in the graph of FIG. 5 will be described. Here, an exercise managing method in a case in which the user having a set target HR of 134 bpm runs will be described. It may be assumed that a total exercise duration suggested to the user is 30 minutes, and a steady state is reached at a point in time at which five minutes passes after an exercise is initiated. The exercise managing apparatus may analyze characteristics of the exercise performed by the user as follows.

(1) Target HR for the user: 134 bpm

(2) Target cumulative HR after steady state is reached = 134 x 25 min x 60 sec = 201,000 beats

The target cumulative HR may be determined by multiplying a target HR by an exercise duration, as expressed by Equation 1, and calculated in second units.

(3) $HR_{steady}$: 137.4 bpm

$HR_{steady}$ denotes an HR in the steady state, and reflects an intensity of the actually performed exercise. When it is assumed that the steady state is reached within five minutes after the user initiates the exercise, the exercise managing apparatus may determine a mean HR calculated in a steady state period within five minutes to be $HR_{steady}$.

(4) Second cumulative HR after steady state is reached = 137.4 x 25 min x 60 sec = 206,100 beats

The exercise managing apparatus may calculate the second cumulative HR after the steady state is reached, based on $HR_{steady}$ and Equation 3, as expressed in the above item (4).

The exercise managing apparatus may calculate a value of $\Delta$HR $Sum_{steady}$ based on the second cumulative HR after the steady state is reached and the target cumulative HR, as expressed by Equation 7.

[Equation 7]

$$\Delta HR\ Sum_{steady} = \text{Second cumulative HR - Target cumulative HR}$$
$$= 206{,}100 - 201{,}000 = 5{,}100 \text{ beats}$$

The exercise managing apparatus may determine an exercise performance excess or shortfall rate based on $\Delta$HR $Sum_{steady}$ determined after the steady state is reached and the target cumulative HR, based on Equation 7. The exercise performance excess or shortfall rate may indicate an excess portion or a shortfall portion of a total quantity of the exercise actually performed by the user with respect to a target exercise quantity. The exercise managing apparatus may calculate an exercise performance excess rate based on a ratio of $\Delta$HR $Sum_{steady}$ after the steady state is reached to the target cumulative HR after the steady state is reached, as expressed by, for example, Equation 8.

[Equation 8]

$$\text{Exercise performance excess rate (\%)} = \Delta\text{HR Sum}_{steady} \div \text{Target cumulative HR} \times 100$$
$$= 5{,}100 \div 201{,}000 \times 100 = 2.5\%$$

The exercise performance excess or shortfall rate may be used as a criterion to determine whether the exercise objective data set for the user is to be updated. For example, when the exercise performance excess rate is higher than a preset target value, the exercise managing apparatus may upgrade an exercise intensity to be applied to a future exercise. Conversely, when the exercise performance excess rate is lower than the preset target value, the exercise managing apparatus may downgrade the exercise intensity to be applied to a future exercise.

FIG. 6 illustrates an example of a method of analyzing characteristics of an exercise based on a change in an HR using an exercise managing apparatus in a case in which a user performs an exercise at various intensities.

When a user performs a complicated combination of exercises having various intensities, an HR may increase or decrease and then reach a steady state in time periods with respect to the intensities of the respective exercises. A graph of FIG. 6 illustrates changes in an intensity of an exercise performed by a user at boundaries of respective exercise periods 610, 620, 630, and 640. At a point in time at which the exercise intensity changes, an HR may increase or decrease until a new steady state is reached.

The exercise managing apparatus may divide the entire exercise period into the plurality of exercise periods 610, 620, 630, and 640 based on the exercise intensities, and determine a first cumulative HR and a second cumulative HR with respect to each of the exercise periods 610, 620, 630, and 640. The exercise managing apparatus may determine a first cumulative HR with respect to the entire exercise period by summing up first cumulative HRs of the respective exercise periods 610, 620, 630, and 640, or determine the first cumulative HR with respect to the entire exercise period by continuously accumulating HRs measured in the entire exercise period. The exercise managing apparatus may determine a second cumulative HR with respect to the entire exercise period by summing up second cumulative HRs of the respective exercise periods 610, 620, 630, and 640. The exercise managing apparatus may analyze characteristics of the exercise performed by the user based on the first cumulative HR and the second cumulative HR determined with respect to the entire exercise period, and provide a result of the analyzing to the user.

For example, when an exercise intensity changes at a predetermined point in time while the user is exercising, the exercise managing apparatus may determine an excess portion or a shortfall portion of the quantity of the exercise performed by the user with respect to a target exercise quantity based on a target cumulative HR after a steady state set in the corresponding exercise quantity is reached and a first cumulative HR actually measured after the steady state is reached. The exercise managing apparatus may calculate a value of $HR_{deficit}$ with respect to the entire exercise period by summing up values of $HR_{deficit}$ corresponding to areas 615, 625, and 635 and subtracting a value of $HR_{deficit}$ corresponding to an area 645. The area 645 may be a recovery area in which an exercise intensity decreases to be lower than an intensity of the exercise performed in the previous exercise period 630. The value of $HR_{deficit}$ corresponding to the area 645 may be excluded from the value of $HR_{deficit}$ with respect to the entire exercise period.

FIGS. 7A and 7B illustrate an example of an exercise managing apparatus displaying a result of evaluating exercise effects.

The exercise managing apparatus may manage an exercise activity of a user using exercise contents, and the user may efficiently perform an exercise through the exercise contents. The exercise contents may be specifically designed for the user. The user may receive exercise objectives set for the user, as shown in FIG. 7A, before an exercise is initiated. The user may receive the exercise objectives such as, for example, a target exercise quantity, a target exercise duration, and a target exercise intensity. The exercise intensity may be continuously fixed or variable during the exercise duration based on a selection of the user.

When the user initiates an exercise, the exercise managing apparatus may analyze the exercise performed by the user based on a change in an HR measured at a body of the user, and may display a result of the analyzing in real time. For example, the exercise managing apparatus may display a current cumulative HR determined based on HR information until a current point in time at which the user is performing the exercise, an exercise performance excess or shortfall rate until the current point in time, and an anaerobic/aerobic metabolism rate, as shown in FIG. 7B. In addition, the exercise managing apparatus may display a target HR and a target cumulative HR set for the user along with an HR currently measured on a display screen. The user may verify a change in an exercise state of the user based on the displayed information.

FIG. 8 is a flowchart illustrating an example of an exercise managing method.

Referring to FIG. 8, an exercise managing apparatus measures HRs of a user in operation 810. The exercise managing apparatus may measure HR signals at a body of the user using an EMG sensor or an HR measuring sensor configured to sense potential signals from the surface of the body of the user.

In operation 820, the exercise managing apparatus determines a cumulative HR indicating a cumulative sum of HRs based on the HR information of the user. The exercise managing apparatus may determine a first cumulative HR based on actual HRs measured from a start time of an exercise. For example, the exercise managing apparatus may determine the first cumulative HR by continuously accumulating the HRs from the start time of the exercise to an end time of the exercise, as expressed by Equation 2.

In addition, the exercise managing apparatus may determine a second cumulative HR based on an HR in a steady state. For example, the exercise managing apparatus may determine the second cumulative HR based on a mean HR in a steady state period and an exercise duration, as expressed by Equation 3.

In operation 830, the exercise managing apparatus analyzes characteristics of the exercise performed by the user based on the cumulative HR determined in operation 820. The exercise managing apparatus may determine whether a preset exercise objective is achieved as a result of performing the exercise determined based on the cumulative HR. For example, the exercise objective may include at least one of an exercise duration, an exercise intensity, and an exercise quantity.

The exercise managing apparatus may determine a ratio of anaerobic metabolism to aerobic metabolism used during the exercise of the user based on the first cumulative HR and the second cumulative HR. The exercise managing apparatus may determine the ratio of the anaerobic metabolism used in a time period during which the exercise is performed based on a difference between the second cumulative HR and the first cumulative HR.

The exercise managing apparatus may determine a total quantity of the exercise performed by the user based on the first cumulative HR. In addition, the exercise managing apparatus may determine a result of evaluating the exercise, for example, an exercise performance excess or shortfall rate, based on the second cumulative HR after the steady state is reached and a target cumulative HR. For example, the exercise managing apparatus may determine the exercise performance excess or shortfall ratio based on a difference between the second cumulative HR after the steady state is reached and the target cumulative HR.

In operation 840, the exercise managing apparatus provides a result of the analyzing to the user. The result may be provided via an output device. The exercise managing apparatus may, for example, display the first cumulative HR on a display screen until a current point in time of the exercise, the exercise performance excess or shortfall rate, and the usage ratio of the anaerobic metabolism to the aerobic metabolism. The exercise managing apparatus may provide the overall result of analyzing the exercise to the user, and update exercise objective data set for the user after the user ends the exercise. For example, the exercise managing apparatus may upgrade or downgrade an exercise objective for an exercise to be performed by the user in the future, based on whether the exercise objective is achieved as the result of performing the exercise. As described above, the exercise managing apparatus may analyze an exercise of a user based on HR information, and provide the user with an optimized exercise objective or exercise program suitable for a current state of the user.

The various modules, elements, and methods described above may be implemented using one or more hardware components, one or more software components, or a combination of one or more hardware components and one or more software components.

A hardware component may be, for example, a physical device that physically performs one or more operations, but is not limited thereto. Examples of hardware components include resistors, capacitors, inductors, power supplies, frequency generators, operational amplifiers, power amplifiers, low-pass filters, high-pass filters, band-pass filters, analog-to-digital converters, digital-to-analog converters, and processing devices.

A software component may be implemented, for example, by a processing device controlled by software or instructions to perform one or more operations, but is not limited thereto. A computer, controller, or other control device may cause the processing device to run the software or execute the instructions. One software component may be implemented by one processing device, or two or more software components may be implemented by one processing device, or one software component may be implemented by two or more processing devices, or two or more software components may be implemented by two or more processing devices.

A processing device may be implemented using one or more general-purpose or special-purpose computers, such as, for example, a processor, a controller and an arithmetic logic unit, a digital signal processor, a microcomputer, a field-programmable array, a programmable logic unit, a microprocessor, or any other device capable of running software or executing instructions. The processing device may run an operating system (OS), and may run one or more software applications that operate under the OS. The processing device may access, store, manipulate, process, and create data when running the software or executing the instructions. For simplicity, the singular term "processing device" may be used in the description, but one of ordinary skill in the art will appreciate that a processing device may include multiple processing elements and multiple types of processing elements. For example, a processing device may include one or more processors, or one or more processors and one or more controllers. In addition, different processing configurations are possible, such as parallel processors or multi-core processors.

A processing device configured to implement a software component to perform an operation A may include a processor programmed to run software or execute instructions to control the processor to perform operation A. In addition, a processing device configured to implement a software component to perform an operation A, an operation B, and an operation C may have various configurations, such as, for example, a processor configured to implement a software component to perform operations A, B, and C; a first processor configured to implement a software component to perform operation A, and a second processor configured to implement a software component to perform operations B and C; a first processor configured to implement a software component to perform operations A and B, and a second processor configured to implement a software component to perform operation C; a first processor configured to implement a software component to perform operation A, a second processor configured to implement a software component to perform operation B, and a third processor configured to implement a software component to perform operation C; a first processor configured to implement a software component to perform operations A, B, and C, and a second processor configured to implement a software component to perform operations A, B, and C, or any other configuration of one or more processors each implementing one or more of operations A, B, and C. Although these examples refer to three operations A, B, C, the number of operations that may implemented is not limited to three, but may be any number of operations required to achieve a desired result or perform a desired task.

Functional programs, codes, and code segments for implementing the examples disclosed herein can be easily constructed by a programmer skilled in the art to which the examples pertain based on the drawings and their corresponding descriptions as provided herein.

Software or instructions for controlling a processing device to implement a software component may include a computer program, a piece of code, an instruction, or some combination thereof, for independently or collectively instructing or configuring the processing device to perform one or more desired operations. The software or instructions may include machine code that may be directly executed by the processing device, such as machine code produced by a compiler, and/or higher-level code that may be executed by the processing device using an interpreter. The software or instructions and any associated data, data files, and data structures may be embodied permanently or temporarily in any type of machine, component, physical or virtual equipment, computer storage medium or device, or a propagated signal wave capable of providing instructions or data to or being interpreted by the processing device. The software or instructions and any associated data, data files, and data structures also may be distributed over network-coupled computer systems so that the software or instructions and any associated data, data files, and data structures are stored and executed in a distributed fashion.

While this disclosure includes specific examples, it will be apparent to one of ordinary skill in the art that various changes in form and details may be made in these examples without departing from the scope of the claims. The examples described herein are to be considered in a descriptive sense only, and not for purposes of limitation.

## Claims

**1.** An exercise managing method comprising:

determining (820), with a processor, a cumulative heart rate, HR, corresponding to a cumulative sum of HRs of a user, wherein the cumulative HR is based on HRs during a steady state, wherein the cumulative HR is determined based on a mean HR in the steady state period and a duration of an exercise performed by the user;
analyzing (830) the exercise performed by the user based on the cumulative HR, wherein the analyzing (830) comprises:

determining a ratio of anaerobic metabolism to aerobic metabolism used by the user during a time period of the exercise, based on a difference between the cumulative HR and another cumulative HR, wherein the another HR is calculated based on HRs measured from a start time of the exercise performed by the user; or
determining an exercise performance excess or shortfall ratio based on a difference between the cumulative HR and a target cumulative HR for the user, wherein the target cumulative HR is determined based on a target HR for the user and the duration of the exercise; and

providing (840) a result of the analyzing to the user.

**2.** The method of claim 1, wherein the determining (820) comprises determining the another cumulative HR by continuously accumulating HRs of the user from the start time of the exercise.

**3.** An exercise managing apparatus (310) comprising:

a heart rate, HR, measurer configured to measure a change in an HR of a user;
an exercise manager (330) configured to:

determine a cumulative HR in an exercise period of the user based on a mean HR in a steady state period and a duration of an exercise performed by the user, and
analyze said exercise based on the determined cumulative HR, comprising:

determining a ratio of anaerobic metabolism to aerobic metabolism used by the user during a time period of the exercise, based on a difference between the cumulative HR and another cumulative HR, wherein the another HR is calculated based on HRs measured from a start time of the exercise performed by the user; or
determining an exercise performance excess or shortfall ratio based on a difference between the cumulative HR and a target cumulative HR for the user, wherein the target cumulative HR is determined based on a target HR for the user and the duration of the exercise; and

a display unit configured to display a result of the analyzing.

**4.** The apparatus of claim 3, wherein the exercise manager (330) is configured to determine that the HR reaches steady state in response to the change in the HR of the user or an elapsed time of the exercise satisfying a predetermined condition.

**5.** A non-transitory computer-readable storage medium storing instructions to cause a computer to perform the method of claim 1 or 2.

**Patentansprüche**

**1.** Übungsverwaltungsverfahren, das umfasst:

Bestimmen (820) mit einem Prozessor einer kumulativen Herzfrequenz HR (Heart Rate) entsprechend einer kumulativen Summe von Herzfrequenzen eines Benutzers, wobei die kumulative Herzfrequenz auf Herzfrequenzen während eines stabilen Zustands basiert, wobei die kumulative Herzfrequenz auf der Basis einer durchschnittlichen Herzfrequenz in der Periode des stabilen Zustands und einer Dauer einer vom Benutzer durchgeführten Übung bestimmt wird;
Analysieren (830) der vom Benutzer durchgeführten Übung auf der Basis der kumulativen Herzfrequenz, wobei das Analysieren (830) umfasst:

Bestimmen eines Verhältnisses von anaerobem Stoffwechsel zu aerobem Stoffwechsel, der vom Benutzer während eines Zeitraums der Übung genutzt wird, auf der Basis einer Differenz zwischen der kumulativen Herzfrequenz und einer weiteren kumulativen Herzfrequenz, wobei die weitere Herzfrequenz auf der Basis von Herzfrequenzen berechnet wird, die ab einer Startzeit der vom Benutzer durchgeführten Übung gemessen werden; oder
Bestimmen eines Überschuss- oder Mangelverhältnisses der Übungsleistung auf der Basis einer Differenz zwischen der kumulativen Herzfrequenz und einer kumulativen Sollherzfrequenz für den Benutzer, wobei die kumulative Sollherzfrequenz auf der Basis einer Sollherzfrequenz für den Benutzer und der Dauer der Übung bestimmt wird; und

Bereitstellen (840) eines Ergebnisses der Analyse für den Benutzer.

**2.** Verfahren nach Anspruch 1, wobei das Bestimmen (820) das Bestimmen der weiteren kumulativen Herzfrequenz durch kontinuierliches Akkumulieren von Herzfrequenzen des Benutzers ab dem Startzeitpunkt der Übung umfasst.

**3.** Übungsverwaltungsvorrichtung (310), die umfasst:

einen Herzfrequenzmesser, der konfiguriert ist, um eine Änderung einer Herzfrequenz eines Benutzers zu messen;
einen Übungsmanager (330), der konfiguriert ist zum:

Bestimmen einer kumulativen Herzfrequenz in einer Übungsperiode des Benutzers auf der Basis einer durchschnittlichen Herzfrequenz in einer Periode des stabilen Zustands und einer Dauer einer vom Benutzer durchgeführten Übung, und

Analysieren der Übung auf der Basis der bestimmten kumulativen Herzfrequenz, umfassend:

Bestimmen eines Verhältnisses von anaerobem Stoffwechsel zu aerobem Stoffwechsel, der vom Benutzer während eines Zeitraums der Übung genutzt wird, auf der Basis einer Differenz zwischen der kumulativen Herzfrequenz und einer weiteren kumulativen Herzfrequenz, wobei die weitere Herzfrequenz auf der Basis von Herzfrequenzen berechnet wird, die ab einer Startzeit der vom Benutzer durchgeführten Übung gemessen werden; oder

Bestimmen eines Überschuss- oder Mangelverhältnisses der Übungsleistung auf der Basis einer Differenz zwischen der kumulativen Herzfrequenz und einer kumulativen Sollherzfrequenz für den Benutzer, wobei die kumulative Sollherzfrequenz auf der Basis einer Sollherzfrequenz für den Benutzer und der Dauer der Übung bestimmt wird; und

eine Anzeigeeinheit, die konfiguriert ist, um ein Ergebnis der Analyse anzuzeigen.

**4.** Vorrichtung nach Anspruch 3, wobei der Übungsmanager (330) konfiguriert ist, um zu bestimmen, dass die Herzfrequenz in Reaktion auf die Änderung der Herzfrequenz des Benutzers oder einer abgelaufenen Zeit der Übung, die eine vorgegebene Bedingung erfüllt, einen stabilen Zustand erreicht.

**5.** Nichtflüchtiges, computerlesbares Speichermedium, das Anweisungen speichert, um einen Computer das Verfahren nach Anspruch 1 oder 2 durchzuführen zu lassen.

## Revendications

**1.** Procédé de gestion d’exercice comprenant :

détermination (820), avec un processeur, d’une fréquence cardiaque, FC, cumulée correspondant à une somme cumulée de plusieurs FC d’un utilisateur, dans lequel la FC cumulée repose sur des FC au cours d’un état continu, dans lequel la FC cumulée est déterminée selon une FC moyenne sur la période d’état continu et une durée d’un exercice effectué par l’utilisateur ;

analyse (830) de l’exercice effectué par l’utilisateur selon la FC cumulée, dans lequel l’analyse (830) comprend :

détermination d’un rapport de métabolisme anaérobie sur métabolisme aérobie utilisé par l’utilisateur sur une durée de l’exercice, selon une différence entre la FC cumulée et une autre FC cumulée, dans lequel l’autre FC est calculée selon des FC mesurées à partir d’une heure de début de l’exercice effectué par l’utilisateur ; ou

détermination d’un rapport d’excédent ou de déficit de performances d’exercice selon une différence entre la FC cumulée et une FC cumulée cible pour l’utilisateur, dans lequel la FC cumulée cible est déterminée selon une FC cible pour l’utilisateur et la durée de l’exercice ; et fourniture (840) d’un résultat de l’analyse à l’utilisateur.

**2.** Le procédé de la revendication 1, dans lequel la détermination (820) comprend une détermination de l’autre FC cumulée par une accumulation continue de FC de l’utilisateur depuis l’heure de début de l’exercice.

**3.** Dispositif de gestion d’exercice (310) comprenant :

un module de mesure de fréquence cardiaque, FC, configuré pour mesurer un changement dans une FC d’un utilisateur ;

un gestionnaire d’exercice (330) configuré pour :

déterminer une FC cumulée sur une période d’exercice de l’utilisateur selon une FC moyenne dans une période d’état continu et une durée d’un exercice effectué par l’utilisateur, et

analyser ledit exercice selon la FC cumulée déterminée, comprenant :

détermination d’un rapport de métabolisme anaérobie sur métabolisme aérobie utilisé par l’utilisateur pendant une durée de l’exercice, selon une différence entre la FC cumulée et une autre FC cumulée, dans lequel l'autre FC est calculée selon plusieurs FC mesurées à partir d'une heure de début de l'exercice effectué par l'utilisateur ;
ou
détermination d'un rapport d'excédent ou de déficit de performances d'exercice selon une différence entre la FC cumulée et une FC cumulée cible pour l'utilisateur, dans lequel la FC cumulée cible est déterminée selon une FC cible pour l'utilisateur et la durée de l'exercice ; et
une unité d'affichage configurée pour afficher un résultat de l'analyse.

**4.** Le dispositif de la revendication 3, dans lequel le gestionnaire d'exercice (330) est configuré pour déterminer que la FC atteint un état continu en réponse au changement dans la FC de l'utilisateur ou à une durée écoulée de l'exercice satisfaisant une condition prédéterminée.

**5.** Support de stockage non transitoire lisible par un ordinateur stockant des instructions qui font exécuter à un ordinateur le procédé de la revendication 1 ou 2.

FIG. 1

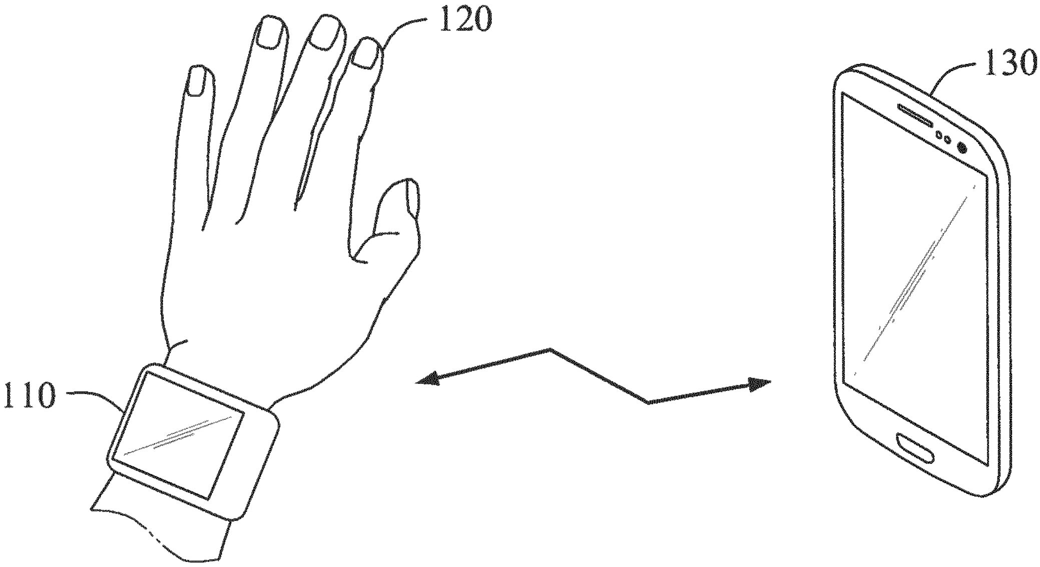

FIG. 2

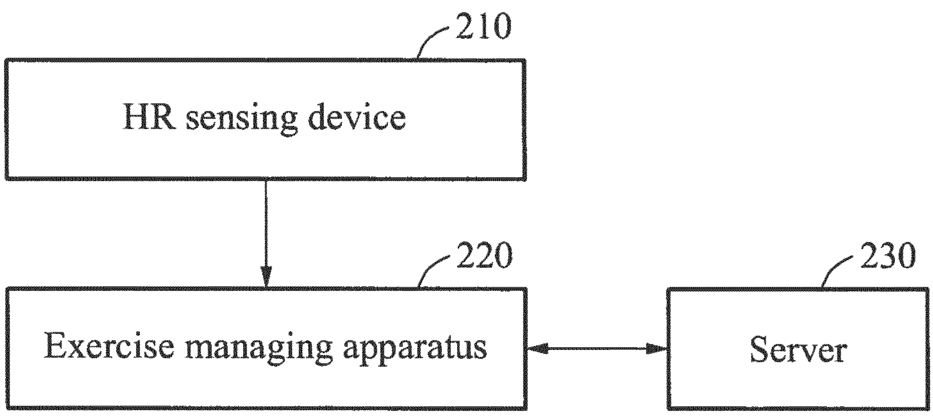
210
HR sensing device
220
Exercise managing apparatus
230
Server

FIG. 3

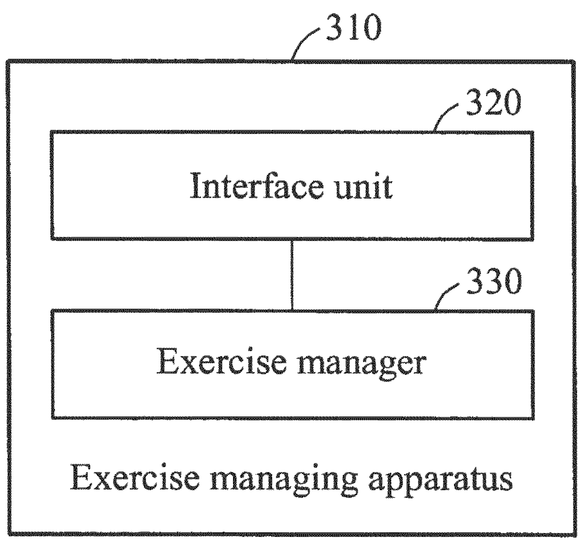
310
320
Interface unit
330
Exercise manager
Exercise managing apparatus

FIG. 4

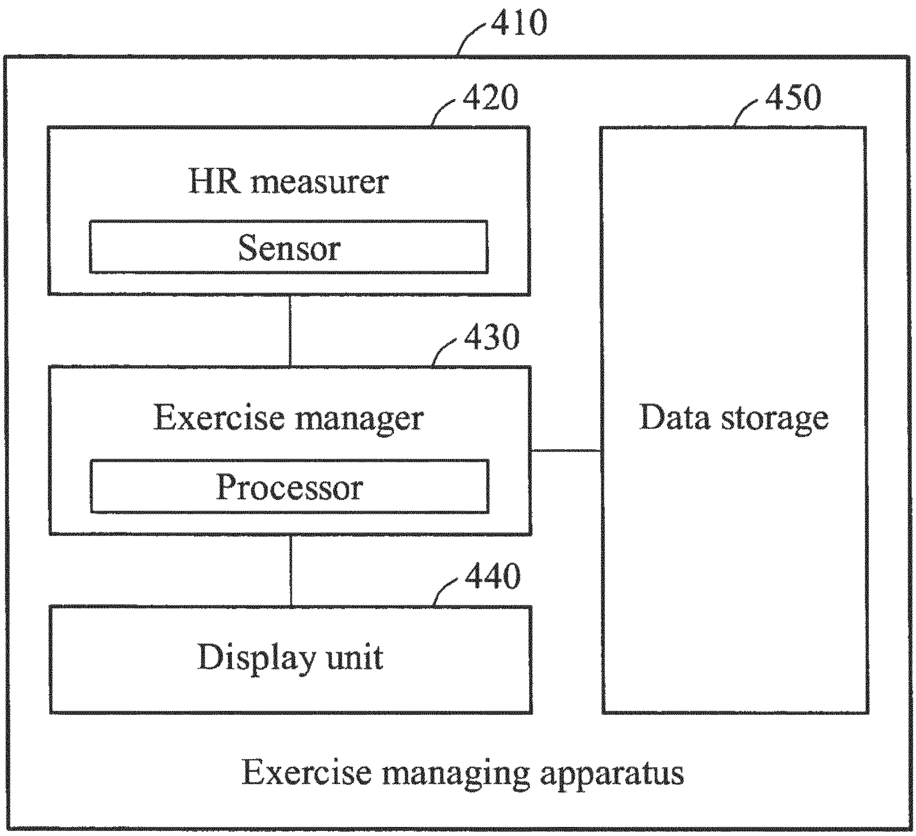
410
420
450
HR measurer
Sensor
430
Exercise manager
Processor
Data storage
440
Display unit
Exercise managing apparatus

FIG. 5

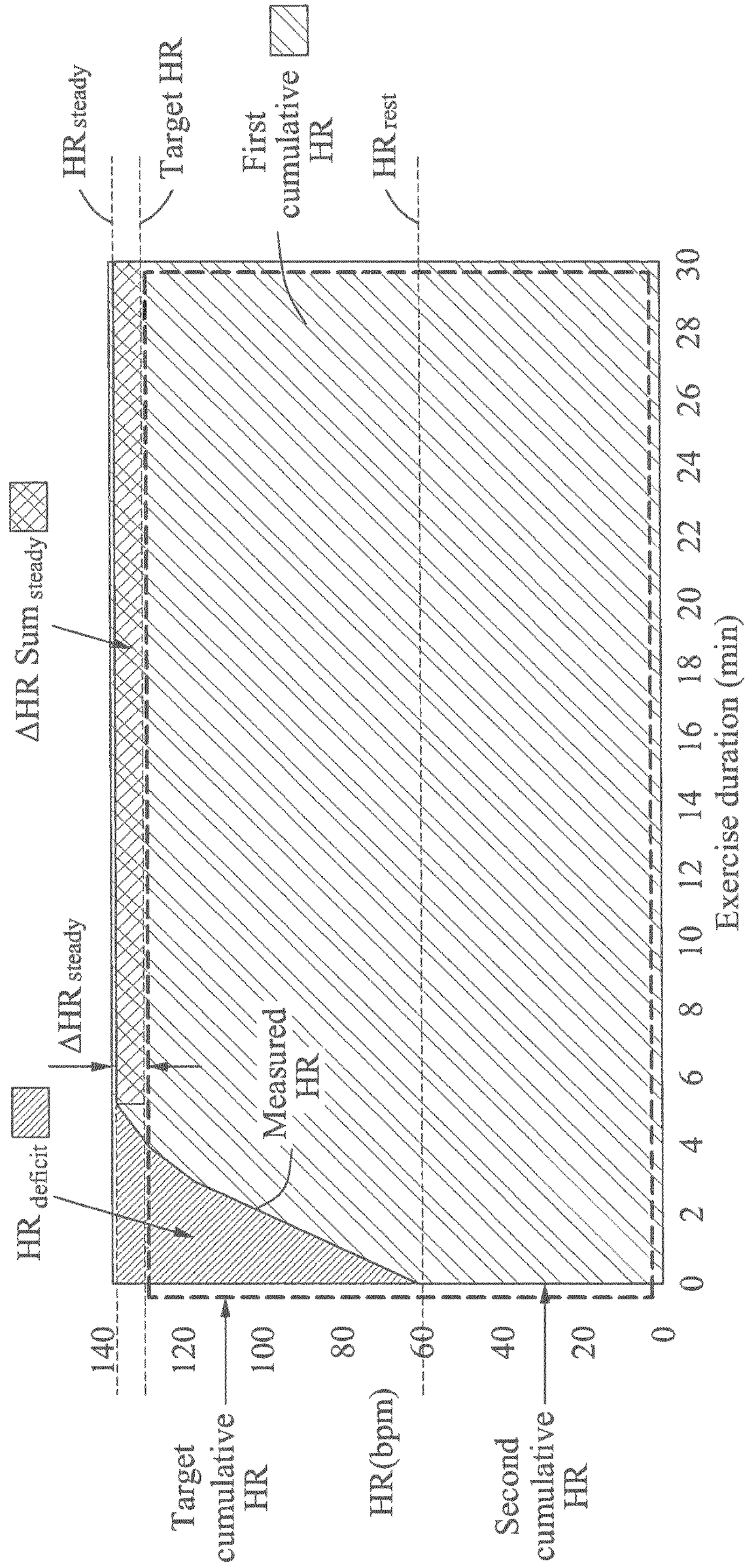
HR steady
Target HR
First cumulative HR
HR rest
ΔHR Sum steady
ΔHR steady
HR deficit
Measured HR
Target cumulative HR
HR(bpm)
Second cumulative HR
140
120
100
80
60
40
20
0
0 2 4 6 8 10 12 14 16 18 20 22 24 26 28 30
Exercise duration (min)

FIG. 6

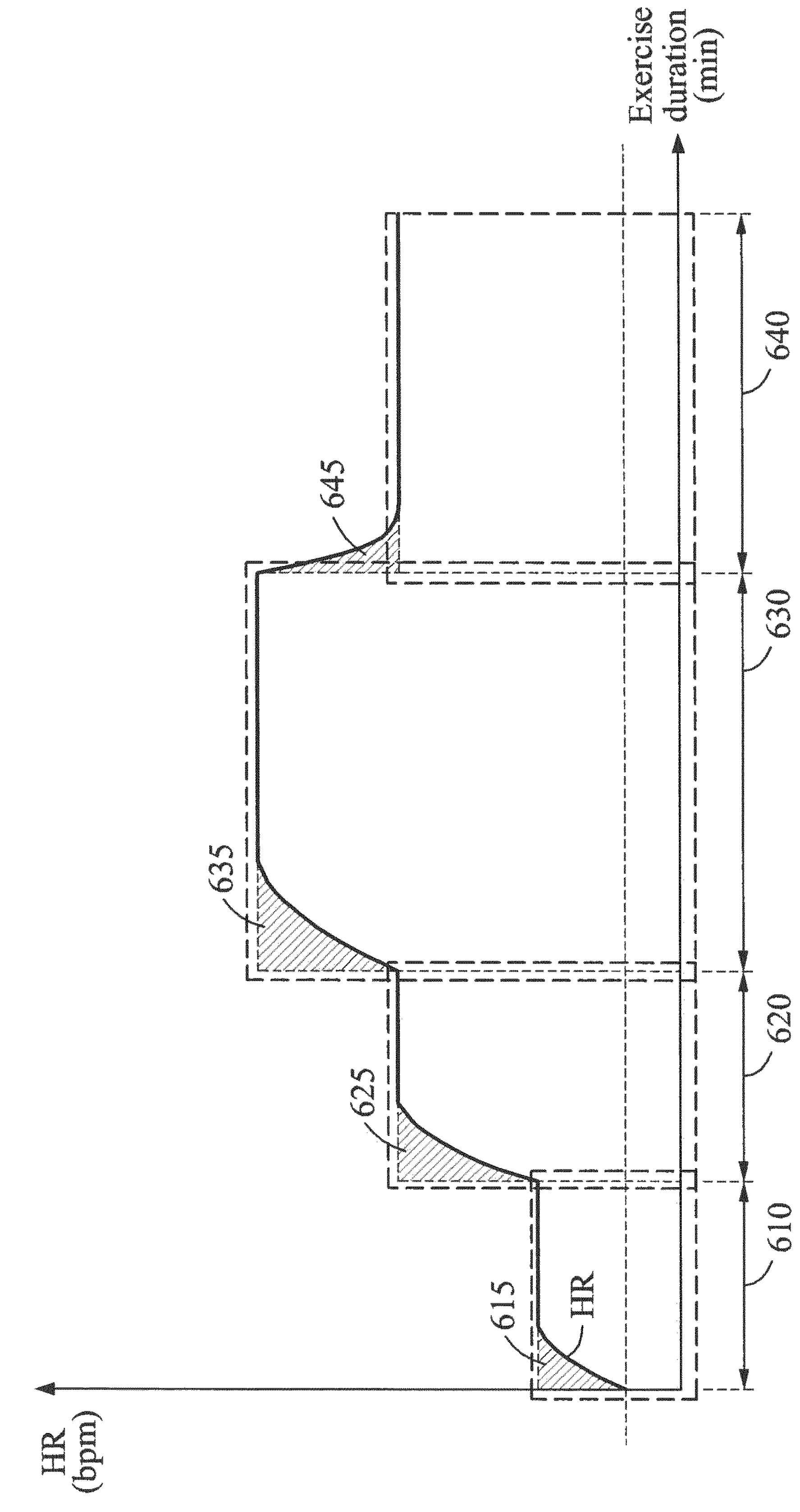
HR (bpm)
Exercise duration (min)
HR
615
625
635
645
610
620
630
640

FIG. 7A

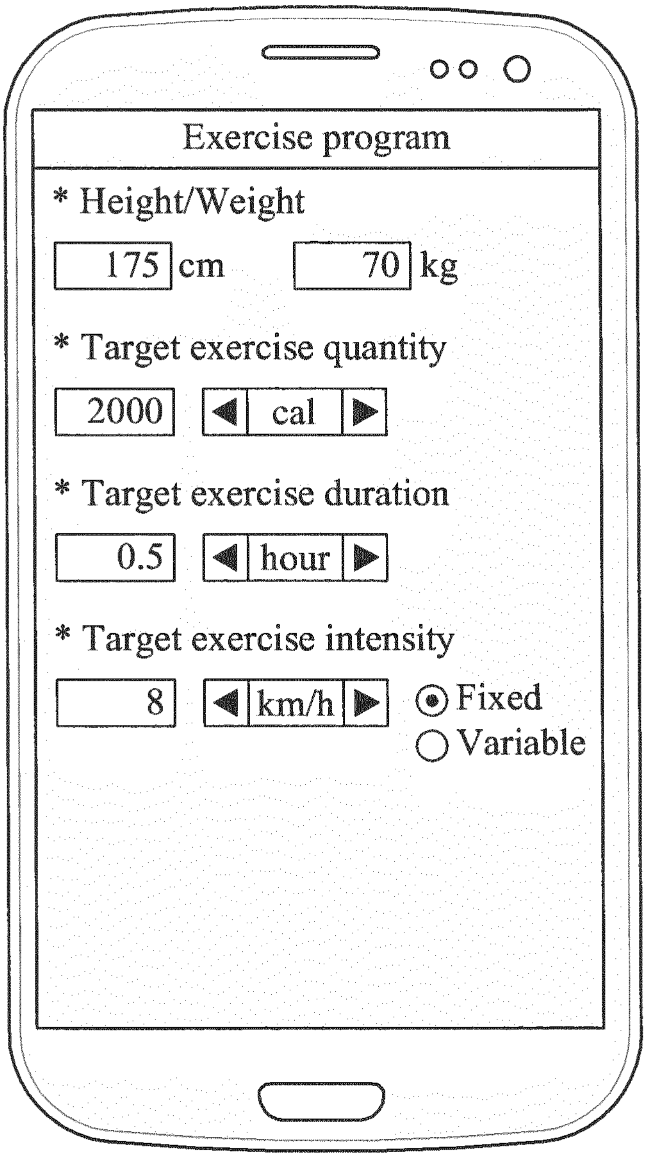
Exercise program
* Height/Weight
175 cm
70 kg
* Target exercise quantity
2000
cal
* Target exercise duration
0.5
hour
* Target exercise intensity
8
km/h
Fixed
Variable

FIG. 7B

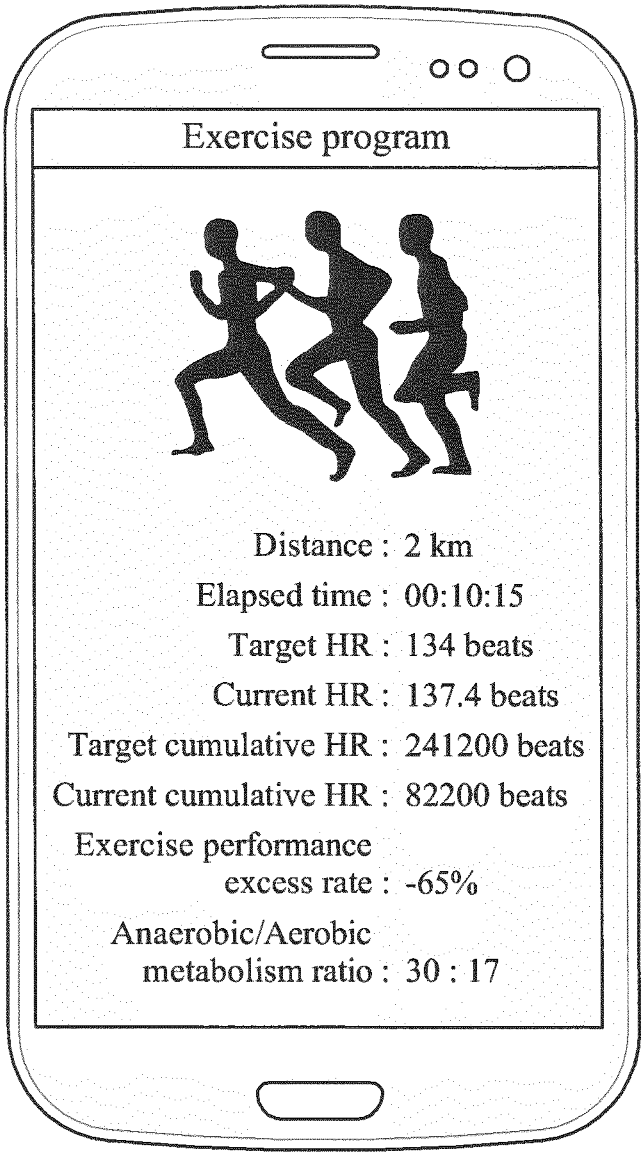
Exercise program
Distance : 2 km
Elapsed time : 00:10:15
Target HR : 134 beats
Current HR : 137.4 beats
Target cumulative HR : 241200 beats
Current cumulative HR : 82200 beats
Exercise performance excess rate : -65%
Anaerobic/Aerobic metabolism ratio : 30 : 17

FIG. 8

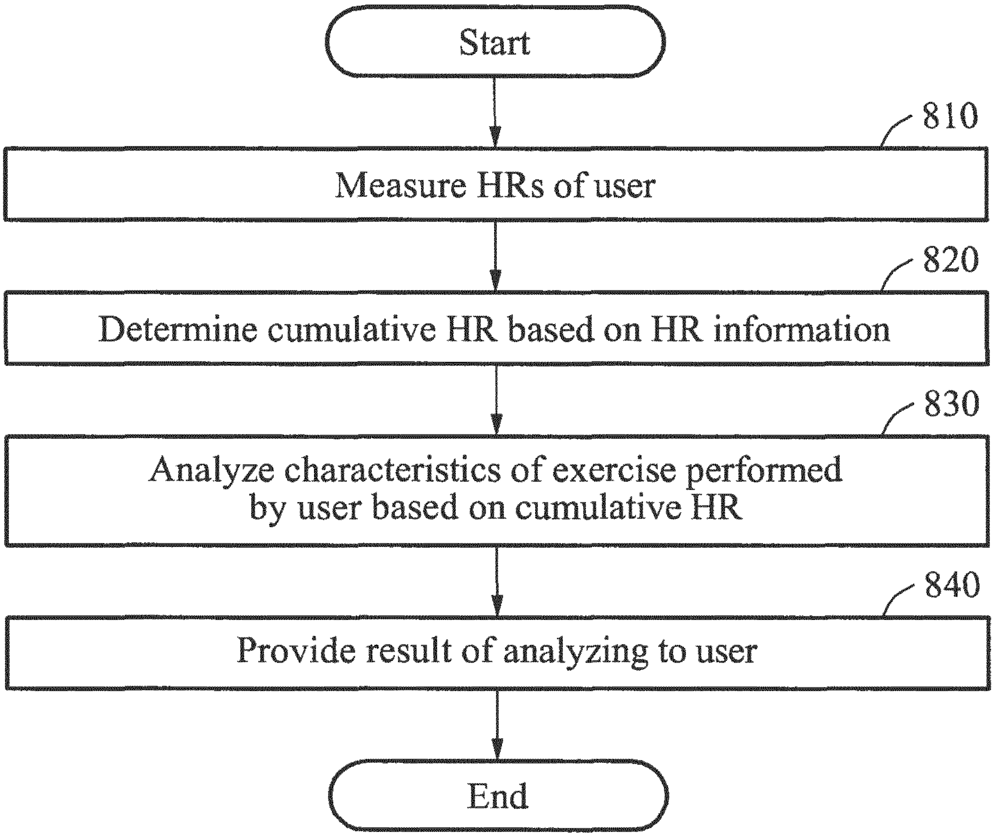
Start
810
Measure HRs of user
820
Determine cumulative HR based on HR information
830
Analyze characteristics of exercise performed by user based on cumulative HR
840
Provide result of analyzing to user
End

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 1369082 A2
- EP 1510175 A1